# EUROPEAN PATENT SPECIFICATION

(11) **EP 0 516 123 B1**
(45) Date of publication and mention of the grant of the patent: **23.04.1997**
(21) Application number: 92109056.9
(22) Date of filing: 29.05.1992
(51) Int. Cl.: A61B 17/068

(54) **Anvilless surgical apparatus for applying surgical fasteners**
Chirurgisches Gerät ohne Amboss zum Anbringen von chirurgischen Befestigern
Dispositif chirurgical sans enclume, pour mettre en place des agrafes chirurgicales

(30) Priority: 30.05.1991 US 707683
(43) Date of publication of application: 02.12.1992
(73) Proprietor: United States Surgical Corporation, Norwalk, Connecticut 06856 (US)
(72) Inventor: Green, David T., Westport, CT 06880 (US); Bolanos, Henry, East Norwalk, CT 06855 (US); Sienkiewicz, Henry R., Stamford, CT 06903 (US); Person, Wayne C., Newtown, CT 06470 (US)
(74) Representative: Marsh, Roy David

(56) References cited:
- EP-A- 0 228 834
- EP-A- 0 324 166
- EP-A- 0 389 102
- EP-A- 0 418 598
- US-A- 1 977 282
- US-A- 4 527 726

## Description

### 1. Field of the Invention

This invention relates to surgical fasteners used in skin grafting and joining of body tissue and, more particularly, to apparatus for applying surgical fasteners composed of a material and configured to work their way out of body tissue after a limited period of time.

### 2. Description of Related Art

Surgical fasteners have been used in operating procedures to eliminate the need for suturing, which is both time consuming and inconvenient. In many applications, the surgeon can use a stapler apparatus, i.e., a fastener implanting device loaded with one or more surgical fasteners, to accomplish in a few seconds what would have taken many minutes to perform by suturing. This reduction in operating time reduces blood loss and trauma to the patient.

A particularly useful procedure for use with surgical fasteners lies in the area of skin grafting. Currently, a wide variety of practitioners, including general surgeons, plastic surgeons, pediatricians and intensive care specialists perform a large percentage of the skin graft procedures done today. Some skin grafts are known as split-thickness grafts since they leave some dermis and are harvested from the patient using a dermatome. This device provides a uniform sheet of skin suitable for grafting. In many cases, the sheet graft is processed through a "meshing" or graft expanding device which makes the graft larger. This is particularly useful in patients experiencing bad burns and who have little healthy skin left after their injury. Meshing can typically expand the surface area of the graft up to three tires its original dimensions.

The remaining types of grafts are full thickness grafts harvested using hand instruments and are typically used in plastic/reconstructive surgery, following trauma, cancer surgery, etc. In full thickness grafts, the entire dermis is removed.

It is preferred that the graft be smoothly and evenly applied to help prevent infection, necrosis, tenting, sloughing, and other complications. This requires that the graft match the underlying topography of the graft site to a high degree. Excessive tension in the graft will result in tenting or non-conformance which can cause graft failure.

In the past, surgical fasteners in the form of metal staples have been used to attach skin grafts. These metal staples are bent by the delivery apparatus to hook together body tissue. Such staples are typically made from biocompatible metals such as stainless steel alloys or titanium. Further, "splinting"_{,} in the form of synthetic grafts, fabric padding, or elastic bandages are also frequently used to insure wound cleanliness and to keep the graft in place. In some instances splinting is sometimes stapled in place over the original stapled graft.

The prior art includes many examples of surgical staplers which do not enclose the body tissue between an anvil and fastener holder. For example, surgical staplers such as those described in U.S. Patent Nos. 3,643,851 and 4,618,086 and EP-A-324166 approach the skin from one direction. However, these staplers require the use of staples which are malleable enough to be crimped by an anvil so that the prongs hook into the tissue. Typically, such staples are made of metal and are not bioabsorbable. They must be removed by another device such as a staple extractor which is not only time consuming but can cause discomfort and pain to the patient. The discomfort and pain in removal of the staples is especially acute when the fasteners are used in skin grafting a burn victim. The sensitivity of a burn patient's skin cannot be understated. Any contact with their skin causes distress let alone removal of fasteners inserted through the skin and embedded in underlying body tissue.

U.S. patent No. 5 108 422, commonly assigned to United States Surgical Corporation, discloses a unique surgical fastener for securing skin grafts. The surgical fastener includes a crown portion and at least two projections which extend from the crown portion, preferably perpendicular to the crown portion and parallel to each other. Each projection includes a tapered tip portion to facilitate penetration into the body tissue and is configured to remain in the body for a relatively short period of time. The material of the projection also has a low coefficient of friction to facilitate ejection from the body tissue. This unique surgical fastener allows for the provision of lateral support across an incision or skin graft interface to provide sufficient lateral force between adjacent tissue sections.

Therefore, it is highly desirable to have an anvilless surgical apparatus for applying surgical fasteners to secure a graft to a layer of skin without causing excess discomfort to the patient.

### SUMMARY OF THE INVENTION

According to the present invention there is provided an anvilless surgical apparatus as claimed in claim 1 below.

The invention makes an anvilless surgical apparatus for applying surgical fasteners to secure grafting material to a desired layer of tissue. The apparatus comprises a housing having a nose portion at a distal end and a handle at its proximal end. A surgical fastener cartridge is disposed in the nose portion and is adapted to receive a plurality of surgical fasteners in longitudinal alignment therein. The cartridge is generally oriented substantially perpendicular to the handle. Driving means is provided within the housing for driving the surgical fasteners through the grafting material and at least partially into the layer of underlying tissue. The driving means is actuable by the handle and generally comprises a pusher rod movable between a distal fired position and a retracted proximal prefired position. Releasing means are also provided in the handle for releasing the pusher rod from its retracted prefired position to cause it to thrust forward with sufficient force to contact a crown portion of a surgical fastener to drive it directly through the grafting material and at least partially into the underlying tissue without forming the projections of the surgical fastener.

A wide variety of surgical fasteners may be used with the present apparatus including both absorbable and non-absorbable type fasteners. However, the fasteners are selected to be suitable for a skin grafting procedure. US-A-4527726 discloses a bone stapler in which a compressed air activated drive means is used to drive metal staples into bone tissue without crimping the staples.

Further, the surgical fastener cartridge may be removable and/or replaceable with a replacement cartridge. It is further envisioned that the nose portion may be rotatable so that the user may pre-set the angular orientation of the cartridge prior to firing.

### BRIEF DESCRIPTION OF THE DRAWINGS

The accompanying drawings, referred to herein and constituting a part hereof, illustrate preferred embodiments of the present invention and, together with the description, serve to explain the principles of the present invention.

FIG. 1 is a perspective view of the present invention showing the handle, nose portion and removable surgical fastener cartridge.

FIG. 2 is a side view in partial cross section of the handle and nose portion of the present invention.

FIG. 3 is a side view in cross section of a fully loaded replaceable cartridge element in accordance with the present embodiment.

FIG. 4 is a side view in cross section of the replaceable cartridge element of FIG. 3 in a fully expended condition.

FIG. 5 is a perspective view of a surgical fastener for use with the present invention.

FIG. 6 is a side view in cross section of an anvilless surgical apparatus in the prefiring position.

FIG. 7 is a side view in cross section of an anvilless surgical apparatus in a position just prior to firing.

FIG. 8 is a side view in cross section of an anvilless surgical apparatus in the fired position.

FIG. 9 is a perspective view in partial cross section of a graft being positioned on tissue.

FIG. 10 is a perspective view in partial cross section of a graft being attached to tissue with an embodiment of the present invention.

### DETAILED DESCRIPTION OF THE PREFERRED EMBODIMENTS

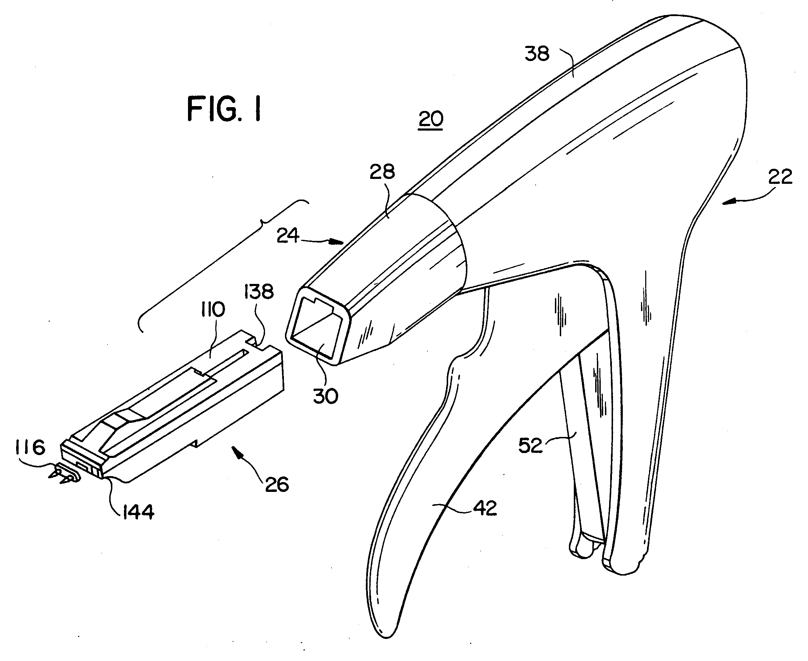

Referring now to the drawings and, in particular, to FIG. 1, there is shown a preferred embodiment of the present anvilless surgical apparatus, shown generally at 20. The apparatus comprises a handle portion 22, a nose portion 24 and a cartridge portion 26. In the preferred embodiment shown in FIG. 1, the nose portion 24 is rotatably mounted to handle portion 22 and is further adapted to receive, in its distal end, an interchangeable cartridge portion 26. One of ordinary skill in the art will readily appreciate that where rotation is not desired nose portion 24 may be integrally formed with handle portion 22. Similarly, where reuse of the handle is not desired, the cartridge portion 26 may be integrally formed and the entire apparatus may be formed as a single use disposable unit.

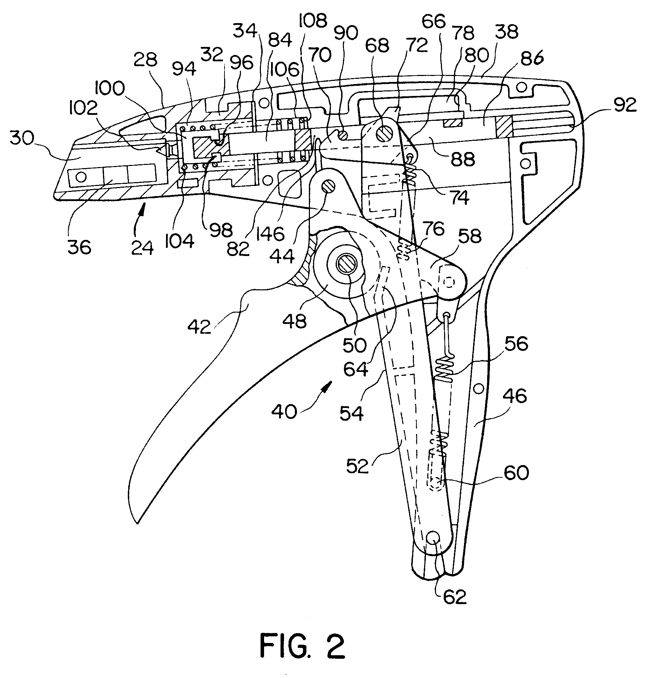

FIG. 2 shows a cross-sectional view of the handle and nose portions 22, 24 of the anvilless surgical apparatus 20 of FIG. 1.

Nose portion 24 includes a housing 28 having a cartridge receiving section 30 in a distal end thereof. The proximal end of housing 28 has a flanged cylindrical element 32 formed therein to interfit with annular groove 34 formed in the distal end of handle portion 22. This allows nose portion 24 to be independently rotatable relative to handle portion 22 and allows the user to select a customized angular orientation for the intended operation. Cartridge receiving section 30 is substantially rectangular in shape and is adapted to receive and retain at least a proximal end of cartridge portion 26. A cartridge retainer spring 36 is disposed within cartridge receiving section 30 and serves to releasably engage cartridge portion 26 when it is in place within the cartridge receiving section 30.

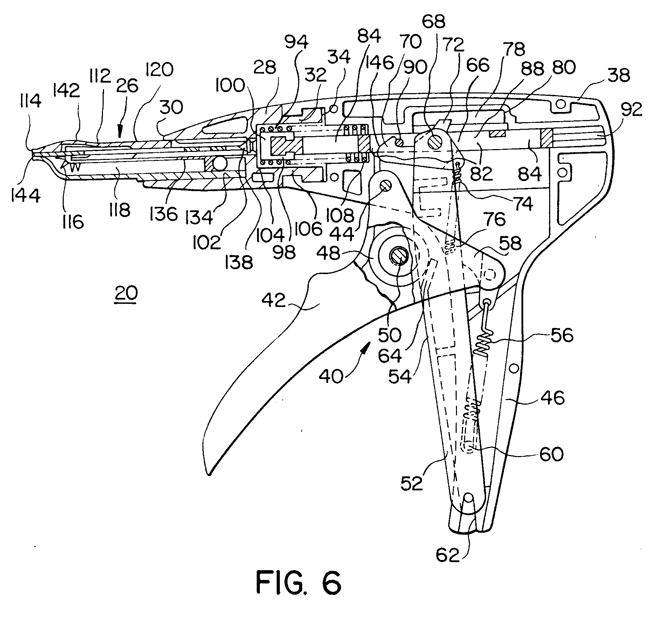

Handle portion 22 includes a housing 38 within which is mounted a firing means shown generally at 40. The firing means 40 has a trigger 42 pivotally mounted in the housing 38 about pivot pin 44. Trigger 42 is adapted for movement between an extended prefired position with trigger 42 distal from hand grip 46 and a compressed fired position with trigger 42 proximal to hand grip 46. This operation will be discussed in greater detail below with respect to FIGS. 6-8. Roller 48 is disposed in trigger 42 and is rotatable therein about pin 50. Roller 48 engages pivot arm 52 and acts against the distal vertical surface 54 thereof as trigger 42 is retracted and released. A trigger return spring 56 is attached at a proximal projection 58 of trigger 42 and extends to fixed pin 60 formed in the hand grip 46 of housing 38. The trigger return spring 56 is in tension and serves to return trigger 42 to its extended distal most position.

Pivot arm 52 extends substantially vertically within hand grip 46 of housing 38 and is pivotal about pin 62. In the present embodiment an angular ramp 64 is formed in distal vertical surface 54 and serves as an action position for roller 48. This ramp 64 is configured to assist the initial pivotal movement of pivot arm 52 by trigger 42. A latch 66 is pivotally mounted in the uppermost portion of pivot arm 52 about pin 68. Latch 66 includes a hook shaped locking member 70 and a vertically oriented tab 72. A latch return spring 74 is connected in tension between latch 66 and fixed pin 76 and serves to maintain a clockwise pull on latch 66 relative to pin 68. A transverse cavity 78 is formed in housing 38 and serves to enclose tab 72 as pivot arm 52 reciprocates about pin 62. As tab 72 engages proximal vertical wall 80 of transverse cavity 78, latch 66 is driven to counterclockwise rotation about pin 68.

A drive shaft 82 extends longitudinally for reciprocal movement within housing 38 and includes a distal end 84, a proximal end 86 and a middle portion 88 therebetween. The middle portion of drive shaft 82 includes a transversely projecting pin 90 which engages hook shaped locking member 70 of latch 66. The proximal end 86 of drive shaft 82 moves longitudinally in channel 92 formed in housing 38. The channel 92 serves to guide drive shaft 82 in its reciprocal longitudinal motion.

The distal end 84 of drive shaft 82 is disposed within chamber 94 formed in housing 38 of the handle portion 22 and housing 28 of the nose portion 24. An annular groove 96 is formed in the distal end 84 of drive shaft 82 and engages projections 98 of tip assembly 100 to securely retain tip assembly 100 onto shaft 82. The tip assembly 100 includes a frustroconical engagement member 102 and a shoulder portion 104. The frustroconical engagement member 102 is accessible through cartridge receiving section 30 as will be described in greater detail below.

A compression spring 106 is disposed in chamber 94 and surrounds the distal end 84 of drive shaft 82. The compression spring 106 is bounded on a distal end by shoulder portion 104 and on a proximal end by shaft spacer washer 108. As the drive shaft 84 moves proximally within housing 38, the proximal movement of shoulder portion 104 serves to compress spring 106 against shaft spacer washer 108 in chamber 94.

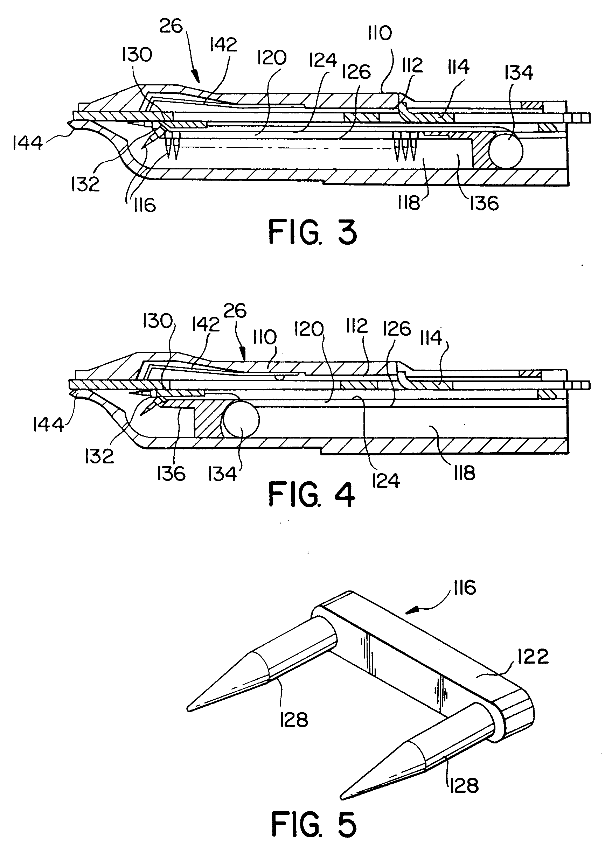

Referring now to FIGS. 3 and 4, there is shown a cartridge portion 26 in accordance with a preferred embodiment of the present invention. Cartridge portion 26 includes a housing 110 with a longitudinal channel 112 formed therein for receiving a driving member 114. A plurality of surgical fasteners 116 (See FIG. 5) are disposed in a longitudinal magazine 118 positioned parallel to longitudinal channel 112. In the embodiment shown in FIGS. 3 and 4, magazine 118 is disposed beneath channel 112, however, one skilled in the art will readily appreciate that the magazine may be disposed above channel 112 if desired.

Magazine 118 includes a longitudinal channel 120 which supports and retains crown portion 122 of surgical fasteners 116 between a top wall 124 and a floor 126. Projections or prongs 128 of the surgical fasteners 116 are initially disposed vertically in the magazine 118. At a distal end of longitudinal channel 120, an angular ramp 130 formed in top wall 124 and floor 126 serves to guide surgical fasteners 116 into a distal vertical portion 132 effectively reorienting the surgical fasteners 116 such that projections 128 are horizontal and projecting distally of the cartridge portion 26. Constant force spring 134 serves to drive rigid follower 136 in abutment with the proximal most surgical fastener to sequentially move the respective distal most surgical fastener into longitudinal channel 112 in preparation for firing. Positioning spring 142 is disposed adjacent vertical portion 132 of channel 120 and serve to hold surgical fasteners 116 in place prior to firing.

Driving member 114 is adapted for reciprocal longitudinal motion within longitudinal channel 112. Locking projections 138 are formed in a proximal end of driving member 114 and, in this embodiment, serve to releasably engage with frustroconical engagement member 102 in a proximal end of cartridge receiving section 30. The distal end of driving member 114 has a substantially flat surface for a smooth interface between the driving member and the crown portion 122 of the surgical fastener 116 as it is fired out of opening 140 at the distal end of longitudinal channel 112. At least one spike 144 is positioned on the distal end of housing 110 in this embodiment to engage tissue and stabilize the apparatus in preparation for firing.

The anvilless surgical apparatus 20 shown and described herein is intended to apply surgical fasteners of the type shown in FIG. 5 and described in detail in corresponding U.S. patent No. 5 108 422, commonly assigned herewith to United States Surgical Corporation. One skilled in the art, however, will readily appreciate that other surgical fastener designs may be used without departing from the inventive features of the present invention.

Referring now to FIGS. 6-8, an anvilless surgical apparatus 20 in accordance with the present invention is shown in a sequence of operation. FIG. 6 shows the apparatus in the prefired position with the cartridge portion 26 in place within the cartridge receiving section 30 of nose portion 24. Locking projections 138 are engaged with frustroconical engagement member 102 such that both driving member 114 and drive shaft 82 move reciprocally longitudinally as a single unit in response to manipulation of the trigger 42. Hook shaped locking member 70 is engaged with transverse pin 90 and shaft 82 is in position to be retracted.

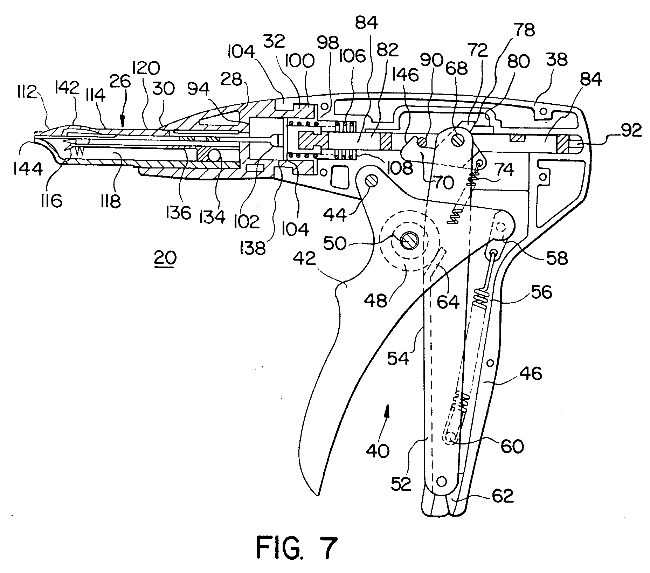

In FIG. 7, trigger 42 is moved proximally toward hand grip 46 causing roller 48 to move pivot arm 52 clockwise relative to pivot pin 62. This motion translates into proximal longitudinal motion of shaft 82 through latch 66 causing driving member 114 to retract in longitudinal channel 112. This motion compresses spring 106 and retracts drive member 114 allowing the distal most surgical fastener 116 in magazine 118 to be pushed into longitudinal channel 112 where it is held by positioning spring 142 in preparation for firing.

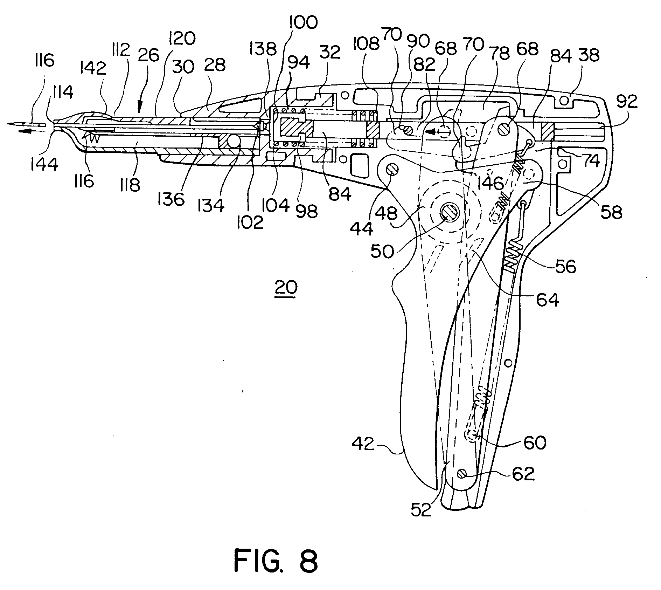

As the trigger 42 continues to move in a proximal direction toward hand grip 46, tab 72 of latch 66 approaches proximal vertical wall 80 of transverse cavity 78. Upon contacting wall 80, continued proximal motion of trigger 42 imparts a counterclockwise motion to latch 66 about pin 68 through tab 72. See FIG. 8. Hook shaped locking member 70 is pivoted out of engagement with pin 90 allowing the drive shaft 82/driving member 114 to be forcefully driven in a distal direction by the expansion of compression spring 106 against shoulder portion 104 of tip assembly 100. The distal end of driving member 114 contacts the crown portion 122 of surgical fastener 116 and drives it out of longitudinal channel 112 into tissue (not shown).

After firing has been completed, trigger return spring 56 and latch return spring 74 return the trigger 42 and latch 66 to their respective prefiring positions. As the latch 66 moves distally toward its original position, cam surface 146 on the distal end of hook-shaped locking member 70 abuts pin 90 and acts to reengage with the shaft 82 for subsequent refiring.

The present embodiment may be continuously refired in this manner until rigid follower 136 has reached the end of the horizontal portion of longitudinal channel 120 just prior to angular ramp 130. At this point, no further surgical fasteners 116 will be moved into horizontal channel 112 for driving. See FIG. 4. If continued applications are necessary, a new cartridge portion 26 may be substituted for the expended cartridge portion.

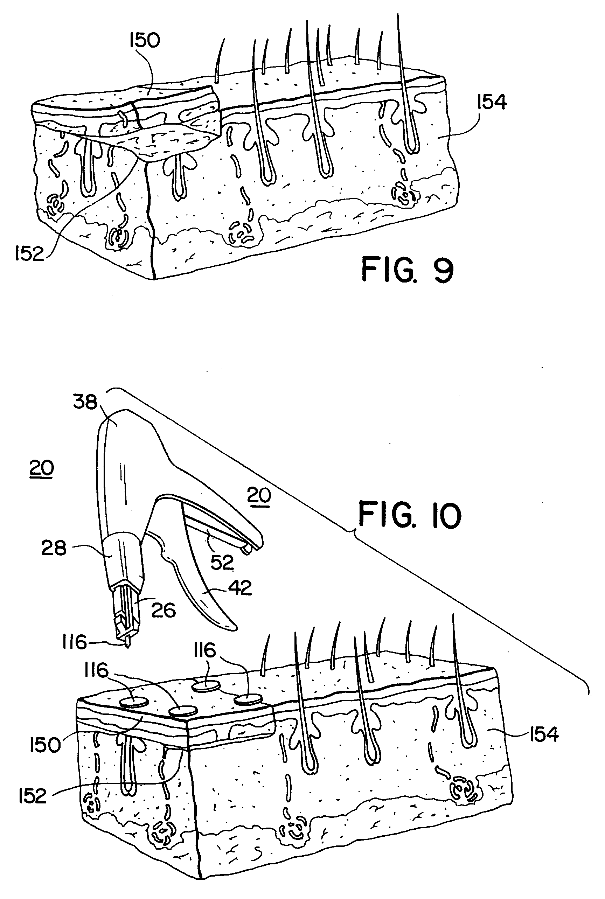

FIGS. 9 and 10 show a split thickness skin graft 150 being applied and fastened into place on surrounding tissue 154. In FIG. 9 the graft 150 is trimmed to cover the damaged skin area 152. Thereafter, the graft is placed over the damaged area 152 and fastened into place using surgical fasteners 116 fired by the anvilless surgical apparatus 20 when the distal end of the magazine of the apparatus is placed in contact with the exposed surface of the graft (preferably at a 90° angle). These fasteners 116 may be applied around the periphery of the graft 150 to secure it adjacent healthy skin and, where desired, may be placed through the graft 150 and into underlying tissue 154.

## Claims

1. Anvilless surgical apparatus (20) for applying surgical fasteners to skin in skin grafting procedures, the apparatus comprising:
a housing (28) having a nose portion (24) at a distal end and a handle portion (22) at a proximal end;
a surgical fastener cartridge (26) longitudinally mounted in said nose portion substantially perpendicular to said handle portion, said cartridge having a plurality of surgical fasteners (116) disposed therein;
means (82) for sequentially driving said surgical fasteners into said graft material and tissue, said means for driving being actuable by a manual manipulation of said handle portion (22) and comprising a drive shaft (82) movable, against the action of a compression spring (106), from a distal fired position to a latched proximal prefired position; and
release means (72) for sequentially firing said drive shaft from said proximal prefired position by permitting the biasing means (106) to thrust the drive shaft forward to contact a crown portion of a leading one of said surgical fasteners to drive said fastener directly into said graft material and tissue without forming the surgical fastener.

2. Apparatus as claimed in claim 1, wherein said nose portion (24) is longitudinally rotatable relative to said handle portion (22).

3. Apparatus as claimed in claim 1 or 2, wherein said surgical fastener cartridge (26) is releasably mounted in said nose portion and replaceable with a new surgical fastener cartridge.

4. Apparatus as claimed in any one of the preceding claims, wherein said surgical fasteners (116) are formed of a polymeric material.

5. Apparatus as claimed in any one of the preceding claims, wherein said surgical fasteners are formed of a bioabsorbable material.

6. Apparatus as claimed in any one of the preceding claims, wherein the handle portion (22) comprises a hand grip (46) and a trigger (42).

7. Apparatus as claimed in any one of the preceding claims wherein: the housing nose portion (24) has a cartridge receiving section formed therein; the surgical fastener cartridge is releasably mounted in said cartridge receiving section (30) of said nose portion, said plurality of surgical fasteners (116) disposed therein being in longitudinal alignment (118) with said nose portion;
the drive shaft is longitudinally reciprocable and spring-loaded; and
actuating means (66) are provided, including said trigger (42), latch means (70) and means for releasing (72) said drive shaft, said release means (72) interacting with said latch means (70) to move said latch means (70) to a release position to permit said forward thrust, and said latch means (70) also serving to retract said drive shaft.

8. Apparatus as claimed in claim 7, wherein said firing means includes a driving member (114) at least partially disposed in said surgical cartridge, said driving member being releasably attached (102, 138) to a distal end of said spring-loaded drive shaft.

9. Apparatus as claimed in claim 7 or 8, wherein said latch means (70) comprises a spring-loaded pivotal latching mechanism (66) engageable with said drive shaft (82) and being driven in longitudinal proximal motion by said trigger.

10. Apparatus as claimed in any one of the preceding claims said surgical fastener cartridge further comprising a rigid follower (136) disposed in abutting relation to a proximal-most surgical fastener in a longitudinal channel positioned parallel to a longitudinal plane formed by said drive shaft.

11. Apparatus as claimed in any one of the preceding claims said surgical fasteners being disposed in said longitudinal channel (120) and having a crown portion and two extending projections, said surgical fasteners being disposed in said channel in abutting end-to-end relation with said projections being oriented perpendicular to the longitudinal plane formed by said drive shaft, said longitudinal channel further comprising an angular ramp (130) interconnecting a vertical section with said longitudinal channel so as to sequentially re-orient said projections of said surgical fasteners in a longitudinal orientation in preparation for firing.

12. Apparatus as claimed in any one of the preceding claims further comprising at least one longitudinally oriented spike (144) disposed in a distal face of said surgical fastener cartridge.

13. Apparatus as claimed in any one of the preceding claims further comprising a positioning spring (142) disposed in said cartridge adjacent the distalmost surgical fastener and adapted to hold said surgical fastener in position for firing.

14. Apparatus as claimed in any one of the preceding claims, wherein said nose portion further includes a cartridge retainer spring (36) oriented to releasably engage said surgical fastener cartridge.

15. Apparatus as claimed in any one of the preceding claims together with grafting material which comprises a synthetic mesh.

## Patentansprüche

1. Anschlaglose chirurgischen Vorrichtung (20) zum Anbringen von chirurgischen Befestigern an Haut in Hauttransplantationsvorgängen, wobei die Vorrichtung umfaßt:
ein Gehäuse (28) mit einem Nasenbereich (24) an einem distalen Ende und einem Griffbereich (22) an einem proximalen Ende;
ein Magazin (26) für chirurgische Befestiger, das in Längsrichtung in dem Nasenbereich im wesentlichen senkrecht zu dem Griffbereich befestigt ist, wobei das Magazin eine Mehrzahl an chirurgischen Befestigern (116) darin angeordnet besitzt;
Mittel (82), um nacheinander die chirurgischen Befestiger in das Transplantatmaterial und Gewebe einzutreiben, wobei das Mittel zum Eintreiben durch eine manuelle Betätigung des Griffbereichs (22) betätigbar ist und umfaßt einen Eintreibschaft (82), der gegen die Wirkung einer Druckfeder (106) von einer distalen abgeschossenen Position zu einer eingeklinkten proximalen Position vor dem Abschießen bewegbar ist; und
Lösemittel (72), um nacheinander den Eintreibschaft von der proximalen Position vor dem Abschießen abzuschießen, indem es dem Vorspannmittel (106) gestattet wird, den Eintreibschaft nach vorne zu schieben, um einen Aufsatzbereich eines vorderen der chirurgischen Befestiger zu berühren, um den Befestiger direkt in das Transplantatmaterial und Gewebe einzutreiben, ohne den chirurgischen Befestiger zu verformen.

2. Vorrichtung gemäß Anspruch 1, worin der Nasenbereich (24) in Längsrichtung relativ zu dem Griffbereich (22) drehbar ist.

3. Vorrichtung gemäß Anspruch 1 oder 2, worin das Magazin (26) für die chirurgischen Befestiger lösbar in dem Nasenbereich befestigt und durch ein neues Magazin für chirurgischen Befestiger ersetzbar ist.

4. Vorrichtung gemäß einem der vorhergehenden Ansprüche, worin die chirurgischen Befestiger (116) aus einem polymerischen Stoff gebildet sind.

5. Vorrichtung gemäß einem der vorhergehenden Ansprüche, worin die chirurgischen Befestiger aus einem bioabsorbierbaren Stoff gebildet sind.

6. Vorrichtung gemäß einem der vorhergehenden Ansprüche, worin der Griffbereich (22) einen Handgriff (46) und einen Abzug (42) umfaßt.

7. Vorrichtung gemaß einem der vorhergehenden Ansprüche, worin: der Nasenbereich (24) des Gehäuses einen Magazinaufnahmeabschnitt darin gebildet besitzt; das Magazin für die chirurgischen Befestiger lösbar in dem Magazinaufnahmeabschnitt (30) des Nasenbereichs befestigt ist, wobei die Vielzahl an chirurgischen Befestigern (116), die darin angeordnet sind, in Längsausrichtung (118) mit dem Nasenbereich sind;
der Eintreibschaft in Längsrichtung hin- und herbewegbar und federbelastet ist; und
Betätigungsmittel (66) vorgesehen sind, umfassend den Abzug (42), ein Fallklinkenmittel (70) und Mittel zum Lösen (72) des Eintreibschaftes, wobei das Lösemittel (72) mit dem Fallklinkenmittel (70) wechselwirkt, um das Fallklinkenmittel (70) in eine Löseposition zu bewegen, um den Schub nach vorne zu gestatten, und das Fallklinkenmittel (70) auch dazu dient, den Eintreibschaft zurückzuziehen.

8. Vorrichtung gemäß Anspruch 7, wobei das Abschießmittel umfaßt ein Eintreibelement (114), das zumindest teilweise in dem chirurgischen Magazin angeordnet ist, wobei das Eintreibelement lösbar an einem distalen Ende des federbelasteten Eintreibschafts angebracht (102, 138) ist.

9. Vorrichtung gemaß Anspruch 7 oder 8, wobei das Fallklinkenmittel (70) umfaßt einen federbelasteten schwenkbaren Fallklinkenmechanismus (66), der mit den Eintreibschaft (82) in Eingriff bringbar ist und in einer längs verlaufenden, proxiamelen Bewegung durch den Abzug angetrieben wird.

10. Vorrichtung gemäß einem der vorhergehenden Ansprüche, wobei das Magazin für die chirurgischen Befestiger weiter umfaßt einen starren Nachfolger (136), der in anstoßender Beziehung zu einem am weitesten proximal gelegenen chirurgischen Befestiger in einer Längsrinne angeordnet ist, die parallel zu einer längs verlaufenden Ebene positioniert ist, die durch den Eintreibschaft gebildet ist.

11. Vorrichtung gemäß einem der vorhergehenden Ansprüche, wobei die chirurgischen Befestiger in der Längsrinne (120) angeordnet sind und einen Aufsatzbereich und zwei sich erstreckende Vorsprünge besitzen, wobei die chirurgischen Befestiger in einer der Rinne in einer anstoßenden Ende-an-Ende-Beziehung mit den Vorsprüngen senkrecht zur Längsebene, die durch den Eintreibschaft gebildet ist, orientiert angeordnet sind, und wobei die Längsrinne weiter umfaßt eine winklige Rampe (130), die einen vertikalen Abschnitt mit der Längsrinne verbindet, um nacheinander die Vorsprünge der chirurgischen Befestiger in einer Längsausrichtung in Vorbereitung für das Abschießen zu reorientieren.

12. Vorrichtung gemäß einem der vorhergehenden Ansprüche, weiter umfassend zumindest eine in Längsrichtung ausgerichtete Spitze (144), die in einer distalen Fläche des Magazins für chirurgischen Befestiger angeordnet ist.

13. Vorrichtung gemäß einem der vorhergehenden Ansprüche, weiter umfassend eine Positionierfeder (142), die in dem Magazin neben dem am weitesten distal gelegenen chirurgischen Befestiger angeordnet ist und dazu geeignet ist, den chirurgischen Befestiger in einer Position für das Abschießen zu halten.

14. Vorrichtung gemäß einem der vorhergehenden Ansprüche, worin der Nasenbereich weiter umfaßt eine Magazinhaltefeder (36), die ausgerichtet ist, um lösbar in Eingriff zu treten mit dem Magazin für chirurgische Befestiger.

15. Vorrichtung gemäß einem der vorhergehenden Ansprüche zusammen mit Transplantatmaterial, das umfaßt ein synthetisches Gewebe.

## Revendications

1. Appareil chirurgical sans enclume (20) pour appliquer des attaches chirurgicales à la peau pendant des procédures de greffe de peau, l'appareil comportant :
un boîtier (28) avec une portion formant nez (24) à l'extrémité distale et une portion de poignée (22) à une extrémité proximale ;
une cartouche d'attaches chirurgicales (26) montée longitudinalement dans ladite portion formant nez sensiblement perpendiculairement à ladite portion de poignée, ladite cartouche comportant une pluralité d'attaches chirurgicales (116) disposées dans celle-ci ;
un moyen (82) pour entraîner séquentiellement lesdites attaches chirurgicales dans ledit matériau de greffe et tissu, ledit moyen d'entraînement étant actionnable par une manipulation manuelle de ladite portion de poignée (22) et comportant un arbre d'entraînement (82) déplaçable, contre l'action d'un ressort de compression (106), d'une position distale après le tir à une position proximale verrouillée avant le tir, et
un moyen de relâchement (72) pour tirer séquentiellement ledit arbre d'entraînement à partir de ladite position proximale avant le tir en permettant au moyen de sollicitation (106) de pousser l'arbre d'entraînement vers l'avant pour venir en contact avec une portion formant couronne d'une attache avant desdites attaches chirurgicales pour entraîner ladite attache directement dans ladite matière de greffe et tissu sans former l'attache chirurgicale.

2. Appareil selon la revendication 1, dans lequel ladite portion formant nez (24) peut tourner longitudinalement relativement à ladite portion de poignée (22).

3. Appareil selon la revendication 1 ou 2, dans lequel ladite cartouche d'attaches chirurgicales (26) est montée relâchablement dans ladite portion formant nez et peut être remplacée par une nouvelle cartouche d'attaches chirurgicales.

4. Appareil selon l'une des revendications précédentes, dans lequel lesdites attaches chirurgicales (116) sont réalisées en une matière polymère.

5. Appareil selon l'une des revendications précédentes, dans lequel lesdites attaches chirurgicales sont réalisées en une matière bio-résorbable.

6. Appareil selon l'une des revendications précédentes, dans lequel la portion de poignée (22) comporte un élément de préhension (46) et un déclencheur (42).

7. Appareil selon l'une des revendications précédentes, dans lequel :
la portion de nez (24) du boîtier comporte une section de réception de cartouche formée dans celle-ci ; la cartouche d'attaches chirurgicales est montée relâchablement dans ladite section de réception de cartouche (30) de ladite portion de nez, ladite pluralité d'attaches chirurgicales (116) disposées dans celle-ci étant en alignement longitudinal (118) avec ladite portion de nez ;
l'arbre d'entraînement peut effectuer un mouvement de va-et-vient longitudinal et est soumis à l'action d'un ressort ; et
des moyens d'actionnement (66) sont prévus, incluant ledit déclencheur (42), un moyen de loquet (70) et un moyen de relâchement (72) dudit arbre d'entraînement, ledit moyen de relâchement (72) agissant avec ledit moyen de loquet (70) pour amener ledit moyen de loquet (70) dans une position de relâchement pour permettre ladite poussée vers l'avant, et ledit moyen de loquet (70) servant également à retirer ledit arbre d'entraînement.

8. Appareil selon la revendication 7, dans lequel ledit moyen de tir comporte un élément d'entraînement (114) disposé au moins partiellement dans ladite cartouche chirurgicale, ledit élément d'entraînement étant attaché relâchablement (102, 138) à une extrémité distale dudit arbre d'entraînement sollicité par ressort.

9. Appareil selon la revendication 7 ou 8, dans lequel ledit moyen de loquet (70) comporte un mécanisme de verrouillage pivotant (66) chargé par ressort pouvant être mis en prise avec ledit arbre d'entraînement (82) et étant entraîné suivant un mouvement proximal longitudinal par ledit déclencheur.

10. Appareil selon l'une des revendications précédentes, où ladite cartouche d'attaches chirurgicales comporte en outre un suiveur rigide (136) disposé en relation de butée avec une attache chirurgicale la plus proche dans un canal longitudinal positionné parallèlement à un plan longitudinal formé par ledit arbre d'entraînement.

11. Appareil selon l'une des revendications précédentes, où lesdites attaches chirurgicales sont disposées dans ledit canal longitudinal (120) et comportent une portion de couronne et deux saillies étendues, lesdites attaches chirurgicales étant disposées dans ledit canal suivant une relation de butée bout-à-bout, lesdites saillies étant orientées perpendiculairement au plan longitudinal formé par ledit arbre d'entraînement, ledit canal longitudinal comportant en outre une rampe angulaire (130) interconnectant une section verticale avec ledit canal longitudinal de façon à réorienter séquentiellement lesdites saillies desdites attaches chirurgicales suivant une orientation longitudinale en préparation du tir.

12. Appareil selon l'une des revendications précédentes, comportant en outre au moins une pointe orientée longitudinalement (144) disposée dans une face distale de ladite cartouche d'attaches chirurgicales.

13. Appareil selon l'une des revendications précédentes, comportant en outre un ressort de positionnement (142) disposé dans ladite cartouche pour être adjacent à l'attache chirurgicale la plus distale et apte à tenir ladite attache chirurgicale en position de tir.

14. Appareil selon l'une des revendications précédentes, dans lequel ladite portion de nez comporte en outre un ressort de retenue de cartouche (36) orienté pour une mise en prise relâchable avec ladite cartouche d'attaches chirurgicales.

15. Appareil selon l'une des revendications précédentes, conjointement avec une matière de greffe qui comporte un filet synthétique.
